Europäisches Patentamt
European Patent Office
Office européen des brevets

(11) Publication number: 0 273 528 A1

(12) **EUROPEAN PATENT APPLICATION**

(21) Application number: 87202595.2

(22) Date of filing: 18.12.87

(51) Int. Cl.⁴: C07C 49/84, C07C 45/68, C07C 149/32, C07C 147/06, C07C 147/14, C07C 143/86, C07C 107/06, C07C 79/36, C07C 97/10, C07D 295/10, C07C 103/76

(30) Priority: 19.12.86 DE 3643403

(43) Date of publication of application: 06.07.88 Bulletin 88/27

(84) Designated Contracting States: AT BE CH DE ES FR GB GR IT LI LU NL SE

(71) Applicant: Shell Agrar GmbH & Co. KG
Binger Strasse 73
D-6507 Ingelheim am Rhein(DE)

(72) Inventor: Curtze, Jürgen
Rheingaublick 6
D-6222 Geisenheim-Johannisberg(DE)

(74) Representative: Hunter, Keith Roger Ian
4 York Road
London SE1 7NA(GB)

(54) **Benzophenones and their preparation.**

(57) Process for the preparation of methoxy benzophenone derivatives of the formula I:

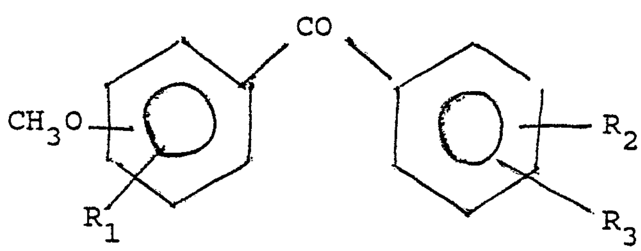

(I)

which comprises reacting an anisole derivative of formula II

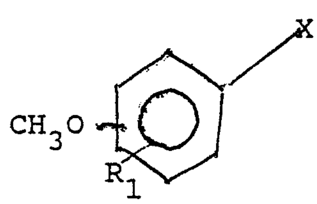

II

with a compound of the formula III

EP 0 273 528 A1

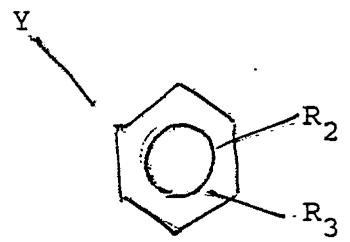

III

wherein one of X and Y represents a hydrogen atom and the other represents the group COCl, or one represents a magnesium halide group MgHal wherein Hal denotes a halogen atom and the other represents COCl or an aldehyde or nitrile group, followed in the last two cases by oxidation or hydrolysis respectively; the substituents $R_1$, $R_2$ and $R_3$ representing a hydrogen or halogen atom or various organic grouping. Certain of the resulting compounds are novel, and are included as such.

# BENZOPHENONES AND THEIR PREPARATION

This invention relates to the preparation of certain substituted benzophenone derivatives, useful inter alia as intermediates in the preparation of agrochemicals, some of which are novel.

Accordingly, the present invention provides a process for the preparation of methoxy benzophenone derivatives of the formula:-

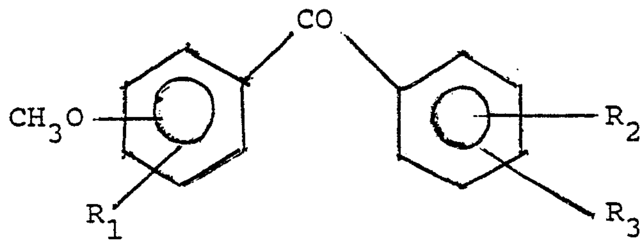

(I)

by reacting an anisole derivative of formula II

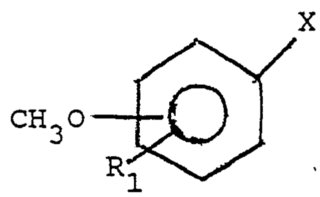

II

with a compound of formula III

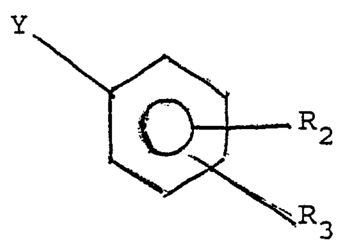

III

wherein one of X and Y represents a hydrogen atom and the other represents the group COCl; or one represents a magnesium halide group MgHal, wherein Hal is a halogen, preferably bromine or iodine, atom, and the other represents COCl or an aldehyde or nitrile group, followed in the last two cases by oxidation or hydrolysis respectively, the substituents $R_1$, $R_2$ and $R_3$ having the following meanings:

$R_1$ represents a hydrogen or halogen atom or an optionally substituted alkyl or alkoxy group; $R_3$ represents a hydrogen or halogen atom, an optionally substituted alkyl or alkoxy group, or together with $R_2$ represents a bivalent group selected from $-(CH_2)_n-$, wherein n is 3, 4 or 5; $-CH=CH-CH=CH-$or

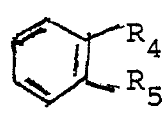

wherein $R_4$ represents O, S, $CH_2$ or $CH_2CH_2$ and $R_5$ represents a covalent bond or, when $R_4$ is $CH_2$ or $CH_2CH_2$ alternatively may represent -O-;
and $R_2$ represents a hydrogen or halogen atom, a nitro or cyano group, an optionally substituted $C_{1-12}$alkyl, $C_{3-8}$ cycloalkyl, $C_{3-12}$ alkenyl, $C_{5-8}$cycloalkenyl, $C_{3-8}$ alkynyl, phenyl, phenylalkenyl, benzyl, $C_{1-12}$ alkoxy, $C_{1-12}$ cycloalkoxy, $C_{3-8}$ alkenyloxy, $C_{3-8}$alkynyloxy, (halo)phenoxy, benzyloxy, $C_{1-12}$ alkyl $S(O)_p$, phenyl $S(O)_p$,

benzyl $S(O)_p$, wherein p=0, 1 or 2, $C_{1-12}$ alkylcarbamoyl, phenylcarbamoyl, $C_{1-12}$ alkylsulphamoyl, phenylazo, phenylamino, benzylamino, acylamino, or a heterocyclic group selected from CO-morpholino, piperidino, pyrrole, pyrazole, imidazole, triazole, dioxolane, and dioxane.

The groupings referred to as "optionally substituted" may contain, as substituent(s), any of those substituents customarily employed in the development of pesticidal compounds, and/or the modification of such compounds to influence their structure/activity, persistence, penetration or other property, for example one or more halogen, nitro, cyano, $C_{1-4}$alkyl or alkoxy groups, preferred such substituents being fluorine, chlorine, bromine, methyl, trifluoromethyl, methoxy. The alkyl and alkoxy groups may be straight chain or branched, and may contain oxygen or sulphur atoms within their chain.

Preferred products are those wherein $R_1$ represents $C_{1-3}$ alkyl or alkoxy, or a chlorine, bromine or iodine atom; $R_3$ represents a hydrogen, fluorine, chlorine or bromine atom or a methyl or methoxy group, and $R_2$ has any of the meanings defined above, especially chlorine, phenyl or halophenoxy.

These starting materials of formula II and III are known products, and may themselves be prepared according to established methods or routine adaptations thereof.

When one of X and Y is hydrogen and the other is COCl, the process is a Friedel Crafts reaction and is effected in the presence of a Lewis acid catalyst according to well-established procedures. Suitable such catalysts include $AlCl_3$, $SnCl_4$, $FeCl_3$, $ZnCl_2$, $TiCl_4$, $SbCl_5$ and $BF_3$, which are normally used in a molar equivalent amount (based on the acyl chloride). However, it is also possible to use lesser amounts of catalyst at elevated temperatures, suitably under reflux, preferred catalysts under these conditions being $FeCl_3$, $I_2$, $ZnCl_2$, iron, copper, strong sulphonic acids such as $F_3C\text{-}SO_3H$, and acidic ion exchange resins such as "Amberlyst" 15 and "Nafion". The reaction can be carried out in a solvent inert under the reaction conditions, for example ethylene or methylene chloride, benzene, decane or solvent mixtures, or in the absence of a solvent, conveniently by employing one of the reactants in excess.

When one of X and Y represents the magnesium halide group MgHal, the process is a Grignard reaction and is effected according to well-established procedures. Thus, bromide and iodide are preferred as the magnesium halide, and the reaction is suitably carried out in solution in an anhydrous ether, such as diethyl, dipropyl or dibutyl ether, anisole or tetrahydrofuran. When the magnesium halide is reacted with an acid chloride, i.e. the other of X or Y represents COCl, the process is conveniently carried out in the presence of ferric tris(acetylacetonate) according to the procedure described in Tetrahedron Letters, 25, No 42, pages 4805-8, and yields directly the desired benzophenone product of formula I.

When the magnesium halide is reacted with a nitrile, i.e. the other group X or Y represents CN, the immediate reaction product is an imine of the formula IV:

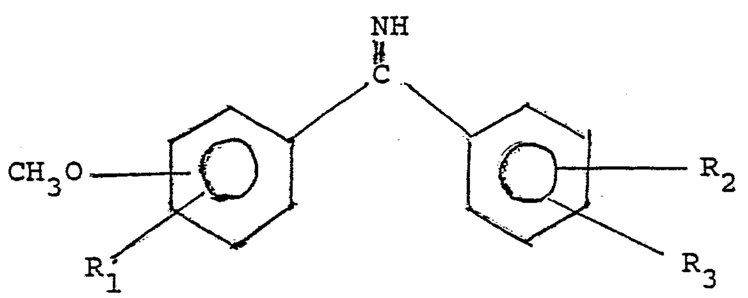

IV

This intermediate is readily converted to the desired benzophenone derivatives of formula I by acid hydrolysis, suitably using mineral acids such as hydrochloric or sulphuric.

When the magnesium halide is reacted with an aldehyde, i.e. the other group X or Y represents CHO, the immediate reaction product is a tertiary alcohol of the formula V:

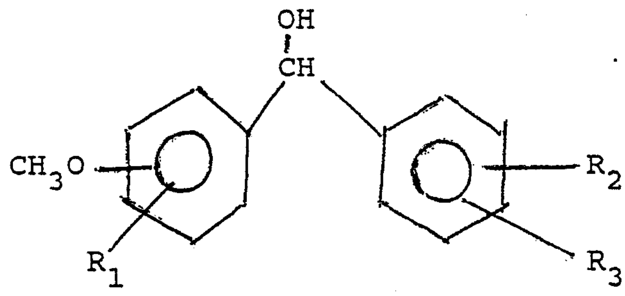

V

This intermediate is readily converted to the desired benzophenone derivatives of formula I by oxidation, suitably using Mn(IV), Mn(VII), Ce(IV) or Cr(VI) derivatives, nitric acid or oxygen in the presence of a catalyst.

The methoxy benzophenones produced according to the process of this invention have a variety of uses, in particular as intermediates in the preparation of pharmaceuticals and fungicides, such as those described in European Patent Application Nos. 84102012 and 86113835.

Certain of these methoxybenzophenones are novel compounds, and as such form a further aspect of this invention. These novel compounds are of formula I defined above, provided that i) $R_2$ and $R_3$ do not both represent a hydrogen atom; ii) when $R_1$ represents a methoxy group, then $R_2$ and $R_3$ do not both represent a chlorine atom or methoxy group, and when $R_2$ represents a hydrogen atom then $R_3$ does not represent a fluorine atom or a methyl or methoxy group; iii) when $R_1$ and $R_3$ both represent a chlorine atom then $R_2$ does not represent a hydrogen atom or a methoxy group; iv) when $R_1$ represents a chlorine atom and $R_3$ represents a hydrogen atom then $R_2$ does not represent a methyl group; and v) when $R_1$ and $R_2$ each represents a methyl group, then $R_3$ does not represent a methoxy group. Preferred novel compounds are those of formula VI

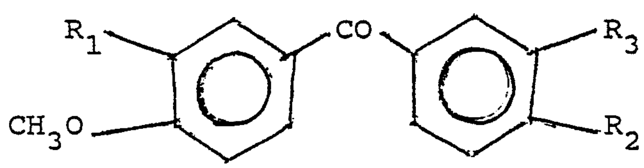

VI

wherein $R_1$ represents a halogen, preferably chlorine or bromine, atom or an alkyl or alkoxy group, suitably containing 1 to 3 carbon atoms; $R_3$ represents a hydrogen or halogen atom or an alkyl or alkoxy group, suitably containing 1 to 8 carbon atoms; and $R_2$ represents a phenyl group or one of the meanings defined for $R_3$; subject to the provisos defined above.

Specific individual novel compounds of particular value include 3,4-dimethoxy-4'-chlorobenzophenone and 3,4-dimethoxy-4'-phenylbenzophenone.

The process of this invention, and the preparation of the novel compounds, are further illustrated in the following examples.

Example I 3,4-dimethoxy-4'-chlorobenzophenone

Procedure A

3.0 mol of veratrol was added to a stirred suspension of 3.0 ml aluminium chloride in 3500 ml of methylene-chloride. 3.0 ml of 4-chlorobenzoylchloride was added to the clear solution and the reaction mixture warmed to reflux temperature. After 4-5 hours under stirring, the reaction mixture was poured into 5 l of ice water. The organic phase was separated and the solvent distilled off under vacuum to yield the desired product in 95% purity and m.pt. 113-114°C.

Procedure B

0.005 mol iron dust was added to a stirred mixture of 0.5 mol veratrole and 0.5 mol 4-chlorobenzoylchloride in 200 ml 110/140 petroleum. The reaction mixture was maintained under reflux for 2-3 hours, filtered hot and cooled to crystallisation in an ice bath, to yield the desired product in 70-80% yield.

Procedure C

138.2 g Veratrol was added to a mixture of 133.5 g aluminium chloride and 1500 ml methylene chloride over 20 minutes at 20-25°C. A clear solution is obtained. Cool this slightly and then drip into it 175.0 g 4-chlorobenzoyl chloride at 20-25°C over a period of 30 minutes. Stir the mixture for 15-18 hours at room temperature then decompose it whilst stirring with 2 litres iced water (1 kg ice and 1 litre water), separate the aqueous phase off and wash with 300 ml methylene chloride. Extract the combined organic phases first with 300 ml 1.5N caustic soda and then twice more with 400 ml water each. After drying over magnesium sulphate, vacuum off and centrifuge off the solvent in a water jet vacuum in the rotary evaporator until a residue is left. The crude produce was extracted with about 500 ml petroleum 80/110, vacuum off, wash with petroleum 80/110 and dry in air.
Yield: 249 g (90% of theory); melting point 109-111°C

Procedure D

Mix together 175 g 4-chlorobenzoyl chloride, 138g Veratrol, 75 g "Amberlyst" 15 and 300 ml decane and boil under reflux for about 2½ hours while stirring. Decent the hot solution off from the catalyst, stir the catalyst with 50 ml hot decane and decant again. Seed the benzophenone solution and cool it slowly while stirring. Draw off the precipitated benzophenone and wash it with 50 ml cold decane.
Yield after drying at 50°C is 250 g crystallised compound (90% of theory); melting point 109-110°C.

Procedure E

0.2 mol (35 g) 4-chloro-benzoylchloride, 0.3 mol (41.4 g) veratrol and 0.5 g copper powder are stirred for 4 h at 200°C while a steady stream of nitrogen is bubbled through the reaction mixture. After cooling down the mixture to 100°C, 200 ml of toluene are added and the hot solution separated from the copper powder by filtration. 200 ml of 5% aqueous sodium hydrogen carbonate are added to the filtrate and the mixture is then stirred at 80°C for 1 h. After separation of the layers the organic layer is washed with 100 ml of water and dried. First the toluene then at 140°C unreacted veratrol (13.85 g) is removed using a rotary evaporator. The crude product is initially obtained as a viscous oil which on treatment with 100 ml of methanol readily crystallises. The mixture is chilled and the product filtered by suction, washed with ice-cold methanol and dried.
Yield: 44.3 g = 80%; mp. 110-111°C.
From mother liquor another 1.05 g = 2% of the product can be isolated.

Example 2

3-ethoxy-4-methoxybenzophenone

a) 3-ethoxy-4-methoxydiphenylcarbinol
Pour a little ether over 3.4 g (0.14 mol) magnesium chips then drip in 22 g (0.14 mol) bromobenzene dissolved in 100 ml abs. ether. After this heat under reflux for ½ hour. Then drip in 20.7 g (0.115 mol) 3-ethoxy-4-methoxybenzaldehyde dissolved in 50 ml abs. ether. Leave to stand for 24 hours, then heat to reflux for another hour. Stir the cooled contents of the flask into water/hydrochloric acid and add a further 200 ml ether. Separate the ether phase and wash twice with water, then dry and concentrate. 26.6 g of the carbinol are obtained.

b) 3-ethoxy-4-methoxybenzophenone

To 6.6 g (0.024 mol) of the carbinol obtained according to a) add 27 ml 10% sulphuric acid, 25 ml glacial acetic acid and then 5 g (0.017 mol) potassium dichromate. Stir the mixture for an hour at 50°C and then add 300 ml water. Separate off the precipitated product, wash with water, then triturate in a little methanol. Renew the methanol twice.

Yield: 4 g; melting point 122-125°C.

Example 3

3,4-dimethoxy-3'-trifluoromethylbenzophenone

Cover 3.65 g (0.15 mol) magnesium chips with 30 ml abs. ether. Dissolve 33.8 g (0.15 mol) 3-bromo-trifluoromethylbenzene in 70 ml abs. ether, then add 10 ml of this solution to the magnesium ether mixture and start the reaction with a grain of iodine. Drip the rest of the 3-bromo-trifluoromethylbenzene in such a way that the reaction keeps going. Then heat to reflux for a $\frac{1}{2}$ hours. Then add 21.2 g (0.13 mol) 3,4-dimethoxybenzonitrile in 200 ml abs. ether in batches. A violent reaction will occur each time. Separate off the solid produced during the reaction. Heat the ether solution to reflux for two more hours, then concentrate it and heat it under reflux together with the previously separated substance with a mixture of ethanol and hydrochloric acid for two hours. Concentrate to dryness and recrystallise the residue out of methanol.

Yield: 21.9 g; melting point 84-86°C

Analysis: Calculated: C: 61.9%; H: 4.19%

Found: C: 61.82%; H: 4.13%

Following procedures similar to those described above, including the use of other Friedel-Crafts catalysts, such as iodine or trifluoromethane sulphonic acid, and also various acidic ion exchangers such as "Lewatite" Sp 1080, "Dowex" 50 w x 4, "Nafion", "Amberlyst" 15, there were synthesised the compounds listed in the Tables below.

TABLE I

Compounds of the formula:

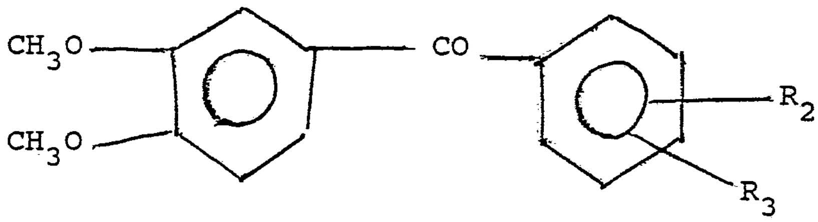

| Compound Number | $R_2$ | $R_3$ | M.Pt. (°) |
|---|---|---|---|
| 1 | 4-Cl | H | 110-112 |
| 2 | 3-Cl | H | 96-98 |
| 3 | 2-Cl | H | 138-141 |
| 4 | 3,4-CH=CH-CH=CH- | | 119-121 |
| 5 | 2-F | H | 84 |
| 6 | 4-$CH_3O$ | H | 98-100 |
| 7 | 4-$CH_3$ | H | 124-126 |
| 8 | 3-Cl | 4-Cl | 92-98 |
| 9 | 3-$CH_3$ | H | 98-101 |
| 10 | 3-$NO_2$ | H | 120-126 |
| 11 | 3-$CH_3O$ | 4-Br | 144-148 |
| 12 | 3-$CH_3O$ | H | 82-84 |
| 13 | 4-Br | H | 116-119 |
| 14 | 4 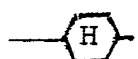 | H | 117-120 |
| 15 | 4 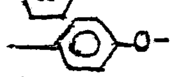 | H | oil |
| 16 | 4-$(CH_3)_2CH$ | H | oil |
| 17 | 4-$C_2H_5$ | H | 103-105 |
| 18 | 4-$C(CH_3)_3$ | H | 85-86 |
| 19 | 2-Cl | 4-Cl | 134-136 |
| 20 | 3-$CH_3$ | 4-$CH_3$ | 88-89 |
| 21 | 3-Cl | 5-Cl | 140-146 |
| 22 (Ex. 3) | 3-$CF_3$ | H | 84-86 |
| 23 | 4-(4-$NO_2$-$C_6H_4$)- | H | 166-169 |
| 24 | 4-$CF_3$ | H | 111-113 |

| Compound Number | $R_2$ | $R_3$ | M.Pt. (°) |
|---|---|---|---|
| 25 | 4-I | H | 118-123 |
| 26 | 3-Cl | 4-F | 119-120 |
| 27 | 4-$C_2H_5O$ | H | 90-95 |
| 28 | 4-n-$C_5H_{11}O$ | H | 85 |
| 29 | 4-$CF_3O$ | H | Resin |
| 30 | 4-$CH_3S$ | H | 113-114 |
| 31 | 4-$CO_2N(CH_3)_2$ | H | 148-151 |
| 32 | 3,4-$CH_2$-$CH_2$-$CH_2$-$CH_2$- | | B.Pt. (0.07mbar) 210-220 |
| 33 | 3,4-$CH_2$-$CH_2$-$CH_2$- | | B.Pt. (0.5mbar) 215-220 |
| 34 | 3,4 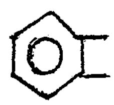 $CH_2$- | | 159-163 |
| 35 | 4-$NO_2$ | H | 167-170 |
| 36 | 4-$C_2H_5S$ | H | 108 |
| 37 | 4-$C_2H_5SO$ | H | 129 |
| 38 | 4-$C_2H_5SO_2$ | H | 141 |
| 39 | 4-(4-Cl-$C_6H_4$-O)- | H | 120-122 |
| 40 | 4-n-$C_5H_{11}$ | H | 70-73 |
| 41 | 4-(4-$CH_3$-$C_6H_4$-O) | H | 108-112 |
| 42 | 4-$CH(CH_3)C_2H_5$ | H | 195-230 |
| 43 | 4-$(CH_3)_2CHCH_2$ | H | B.Pt. (0.07mbar) 195-210 |
| 44 | 4-(4-$C_2H_5$-$C_6H_4$) | H | 102 |
| 45 | 4-n-$C_3H_7$ | H | B.Pt. (0.1mbar) 180-210 |
| 46 | 4-(4-Cl-$C_6H_4$) | H | 116 |

| Compound Number | $R_2$ | $R_3$ | M.Pt. (°) |
|---|---|---|---|
| 47 | $3-(C_6H_5-O)-$ | H | oil |
| 48 | $4-(4-NO_2-C_6H_4-O)$ | H | 159 |
| 49 | $4-n-C_3H_7O$ | H | |
| 50 | $4-n-C_4H_9O$ | H | |
| 51 | $4-OCH_2-CH_2-O-CH_3$ | H | |
| 52 | $3-(4-Cl-C_4H_6-O)$ | H | oil |
| 53 | $4-n-C_5H_{11}$ | H | 59-61 |
| 54 | $4-(2-CH_3-4-Cl-C_6H_3-O)$ | H | 114-116 |
| 55 | $4-(2-Cl-C_6H_4-O)$ | H | 137-139 |
| 56 | $4-(CH_3O-(CH_2)_3)$ | H | oil |
| 57 | $4-(4-CN-C_6H_4-O)$ | H | 149-152 |
| 58 | $4-(3-Cl-C_6H_4-O)$ | H | 80-83 |
| 59 | $4-N=N-C_6H_5$ | H | |
| 60 | $4-CH_3OCH_2$ | H | |
| 61 | $4-n-C_4H_9-NH-CO$ | H | 139-139 |
| 62 | 4-NH-CO 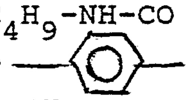 Cl | H | |
| 63 | 4- 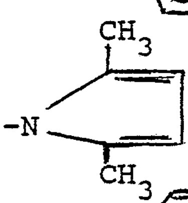 | H | |
| 64 | 4- 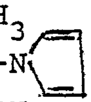 | H | 168-171 |
| 65 | 4-CN | H | 139 |
| 66 | $4-S-CH_2$ 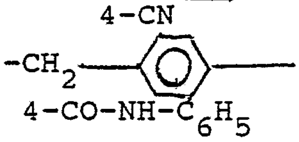 Cl | H | 175-176 |
| 67 | $4-CO-NH-C_6H_5$ | H | 137-138 |
| 68 | $4-CONH-CH_2-C_6H_5$ | H | 194-196 |
| 69 | 3- 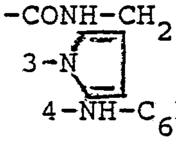 | H | |
| 70 | $4-NH-C_6H_5$ | H | |
| 71 | $4-NH-CH_2-C_6H_5$ | H | |
| 72 | $4-CO-N(CH_3)-n-C_4H_9$ | H | oil |
| 73 | 4 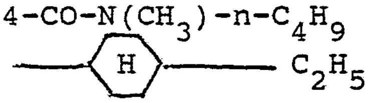 $C_2H_5$ | H | 95-98 |

| Compound Number | R2 | R3 | M.Pt. (°) |
|---|---|---|---|
| 74 | 4 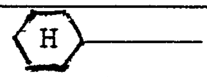 n-$C_7H_{15}$ | H | 95-96 |
| 75 | 3,4 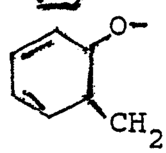 | | 100-103 |
| 76 | 3,4 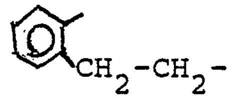 | | 95-105 |
| 77 | 4-n-$C_7H_{15}$ | H | oil |
| 78 | 4-n-$C_8H_{17}$ | H | 64-65 |
| 79 | 3-$CH_3O$ | 4-$CH_3O$ | 144 |
| 80 | 4-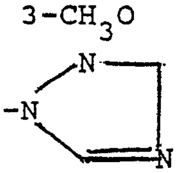 | H | 170-190 |
| 81 | 4-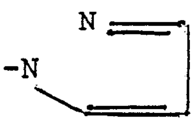 | H | 124-126 |
| 82 | 4-$(CH_3)_2N$-CO- | H | oil |
| 83 | 4-$SO_2$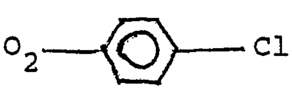 | H | 157-158 |
| 84 | 4-$CH_2O$ 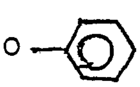 | H | oil |
| 85 | 4-$OCH_2$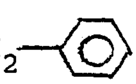 | H | 163 |
| 86 | 4-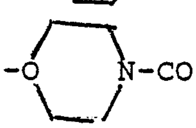 | H | oil |
| 87 | 4-$OCH_2$-CH=$CH_2$ | H | |
| 88 | 4-$OCH_2$-C≡CH | H | |
| 89 | 4-$(CH_3)_2NSO_2O$- | H | |
| 90 | 4-$CF_2HO$- | H | |
| 91 | 4-$C_6H_5(CH_2)_3$-CO-NH- | H | |

| Compound Number | $R_2$ | $R_3$ | M.Pt. (°) |
|---|---|---|---|
| 92 | $3-C_6H_5$ | $4-CH_3O$ | 127-129 |
| 93 | $4-CF_3-CH_2-O$ | H | oil |
| 94 | 4 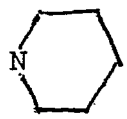 | H | 130-133 |
| 95 | $3-C_6H_5$ | H | |
| 96 | 4 $CHF_2-CF_2-CH_2O-$ | H | oil |
| 97 | 4 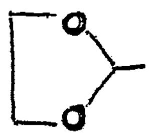 | H | |
| 98 | 4 $C_6H_5-CH=CH-$ | H | |
| 99 | 4 $n-C_3H_7$ 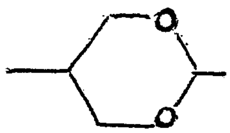 | H | |
| 100 | 4 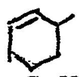 | H | |
| 101 | $4-(4-F-C_6H_4-O)-$ | H | 88-91 |
| 102 | $4-(4-Br-C_6H_4-S)-$ | H | 127-129 |
| 103 | $4-CHFCl-CF_2-O-$ | H | |
| 104 | $4-n-C_3H_7-CH=CH-$ | H | |
| 105 | $4-CHF_2-CF_2-O-$ | H | oil |
| 106 | 4- 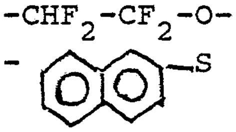 | H | 145-152 |
| 107 | 4 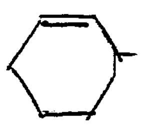 | H | |
| 108 | 4 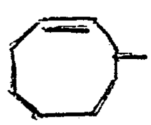 | H | |
| 109 | $4-(C_2H_5O)_2CH$ | H | |
| 110 | $4-(4-Cl-C_6H_4-CH_2)-$ | H | |

| Compound Number | $R_2$ | $R_3$ | M.Pt. (°) |
|---|---|---|---|
| 111 | 4- 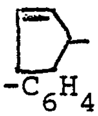 | H | |
| 112 | 4-(4-Cl-$C_6H_4$-O-$CH_2$)- | H | |
| 113 | 4-(4-Cl-$C_6H_4$-S-$CH_2$- | H | |

TABLE 2

Compounds of the formula

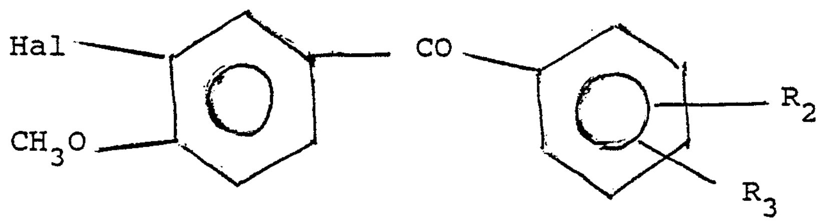

| Compound Number | Hal | $R_2$ | $R_3$ | M.Pt. (°C) |
|---|---|---|---|---|
| 114 | Cl | 4-Cl | H | 115-118 |
| 115 | Cl | H | H | 77-79 |
| 116 | Cl | 3-$CH_3$ | H | 102-104 |
| 117 | Br | H | H | 97 |
| 118 | Br | 3-Br | 4-$CH_3O$ | 173 |
| 119 | Br | 4-$CH_3O$ | H | oil |
| 120 | Br | 3-Cl | 4-$CH_3O$ | 181 |
| 121 | Br | 3-$CH_3$ | 4-$CH_3O$ | 142 |
| 122 | Br | 3-$CH_3$ | 4-$CH_3$ | 88-89 |
| 123 | Br | 4-$CH_3$ | H | 93-98 |
| 124 | Br | 4-Cl | H | 100-112 |
| 125 | Br | 4-n-$C_5H_{11}$ | H | |
| 126 | I | H | H | oil |
| 127 | I | 4-$C_6H_5$ | H | |
| 128 | I | 4-Cl | H | |

TABLE 3

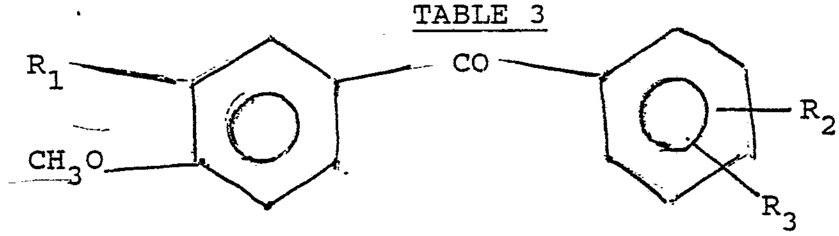

| Compound Number | $R_1$ | $R_2$ | $R_3$ | M.Pt. (°C) |
|---|---|---|---|---|
| 129 | $CH_3$ | H | H | oil |
| 130 | $CH_3$ | $3-CH_3$ | $4-CH_3O$ | oil |
| 131 | $CH_3$ | 4-Cl | H | 112-116 |
| 132 | $CH_3$ | $4-CH_3$ | H | 71-73 |
| 133 | $CH_3$ | $3-CH_3$ | H | 57-61 |
| 134 | $CH_3$ | $4-(CH_3)_3C$ | H | 71-73 |
| 135 | $CH_3$ | 3-Cl | 4-Cl | 100 |
| 136 | $CH_3$ | $4-C_6H_5O$ | H | 58-60 |
| 137 | $CH_3$ | $3-CH_3$ | $4-CH_3$ | 196-197 |
| 138 | $CH_3$ | $4-C_6H_5$ | H | |
| 139 | $3-C_2H_5$ | H | H | 75 |
| 140 | $3-n-C_3H_7$ | H | H | oil |
| 141 | $3-i-C_3H_7$ | H | H | Resin |
| 142 | $3-OC_2H_5$ | H | H | 122-125 |
| 143 | $3-OC_2H_5$ | 4-Cl | H | |
| 144 | H | $4-CH_3O$ | H | 144 |
| 145 | H | $3-CH_3$ | $4-NO_2$ | 115-123 |

## Claims

1. Process for the preparation of methoxy benzophenone derivatives of the formula I:

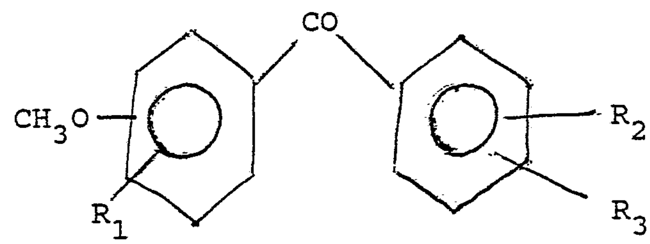

(I)

which comprises reacting an anisole derivative of formula II

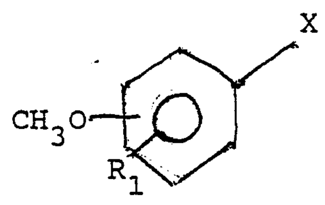

II

with a compound of the formula III

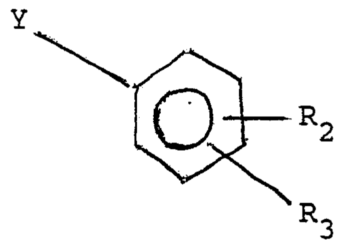

III

wherein one of X and Y represents a hydrogen atom and the other represents the group COCl, or one represents a magnesium halide group MgHal wherein Hal denotes a halogen atom and the other represents COCL or an aldehyde or nitrile group, followed in the last two cases by oxidation or hydrolysis respectively; the substituents $R_1$, $R_2$ and $R_3$ having the following meanings:

$R_1$ represents a hydrogen or halogen atom or an optionally substituted alkyl or alkoxy group; $R_3$ represents a hydrogen or halogen atom, an optionally substituted alkyl or alkoxy group, or together with $R_2$ represents a bivalent group selected from $-(CH_2)_n$ wherein n is 3, 4 or 5, $-CH=CH-CH=CH-$or

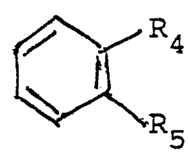

wherein $R_4$ represents O, S, $CH_2$ or $CH_2CH_2$ and $R_5$ represents a covalent bond or when $R_4$ is $CH_2$ or $CH_2CH_2$ alternatively may represent O; and $R_2$ represents a hydrogen or halogen atom, a nitro or cyano group, an optionally substituted $C_{1-12}$alkyl, $C_{3-8}$ cycloalkyl, $C_{3-12}$ alkenyl, $C_{5-8}$cycloalkenyl, $C_{3-8}$ alkynyl, phenyl, phenylalkenyl, benzyl, $C_{1-12}$ alkoxy, $C_{1-12}$cycloalkoxy, $C_{3-8}$ alkenyloxy, $C_{3-8}$ alkynyloxy, (halo)phenoxy, benzyloxy, $C_{1-12}$ alkyl $S(O)_p$ , phenyl $S(O)_p$, benzyl $S(O)_p$, wherein $p=0$, 1 or 2, $C_{1-12}$ alkylcarbamoyl, phenylcarbamoyl, $C_{1-12}$alkylsulphamoyl, phenylazo, phenylamino, benzylamino, acylamino, or a heterocyclic group selected from CO-morpholino, piperidino, pyrrole, pyrazole, imidazole, triazole, dioxolane, and dioxane.

2. Process as claimed in claim 1 wherein one of X and Y is hydrogen, the other represents COCl, and the reaction is effected in the presence of a Lewis-acid catalyst.

3. Process as claimed in claim 2 wherein the catalyst is selected from $AlCl_3$, $SnCl_4$, $FeCl_3$, $ZnCl_2$, $TiCl_4$, $SbCl_5$, $BF_3$, iodine, copper and iron, and the reaction is carried out in an inert organic solvent.

4. Process as claimed in claim 1 wherein one of X and Y is MgHal, wherein Hal represents bromine or iodine, and the reaction is carried out in an anhydrous ether solvent.

5. Process as claimed in claim 4 wherein the other of X and Y represents the group COCl and the reaction is effected in the presence of ferric tris(acetylacetonate).

6. Process as claimed in claim 4 wherein the other of X and Y represents CN, and the intermediate reaction product of formula IV:

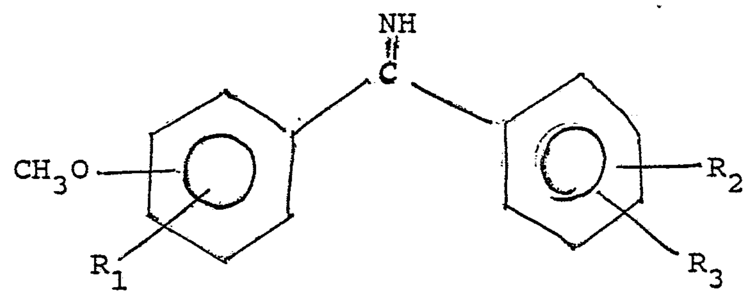

IV

is hydrolysed by a mineral acid.

7. Process as claimed in claim 4 wherein the other of X and Y represents the aldehyde group CHO, and the intermediate reaction product of formula V:

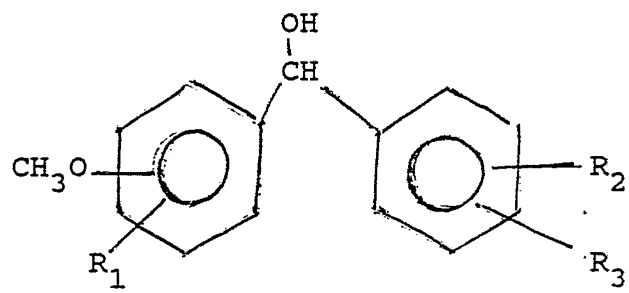

V

is oxidised to a benzophenone of formula I by an oxidant selected from Mn (IV), Mn (VII), Ce (IV), Cr (VI), nitric acid and oxygen in the presence of a catalyst.

8. Methoxybenzophenones of formula I as defined in claim 1 when produced by a process as claimed in any one of the preceding claims.

9. As novel compounds, methoxybenzophenones of the formula I defined in claim 1, provided that i) $R_2$ and $R_3$ do not both represent a hydrogen atom; ii) when $R_1$ represents a methoxy group, then $R_2$ and $R_3$ do not both represent a chlorine atom or methoxy group, and when $R_2$ represents a hydrogen atom then $R_3$ does not represent a fluorine atom or a methyl or methoxy group; iii) when $R_1$ and $R_3$ both represent a chlorine atom then $R_2$ does not represent a hydrogen atom or a methoxy group; iv) when $R_1$ represents a chlorine atom and $R_3$ represents a hydrogen atom then $R_2$ does not represent a methyl group; and v) when $R_1$ and $R_2$ each represents a methyl group, then $R_3$ does not represent a methoxy group.

10. As novel compounds, methoxybenzophenones of the formula VI

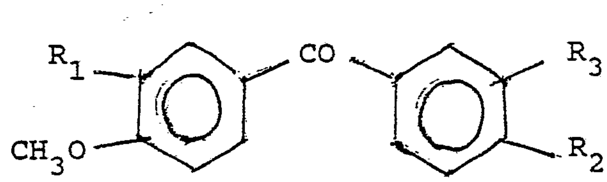

VI

wherein $R_1$ represents a halogen atom or an alkyl or alkoxy group; $R_3$ represents a hydrogen or halogen atom or an alkyl or alkoxy group; and $R_2$ represents a phenyl or halophenoxy group or one of the meanings defined for $R_3$; subject to the provisos defined in claim 9.

11. Compounds as claimed in claim 10 wherein $R_1$ represents a chlorine or bromine atom or a $C_{1-3}$alkyl or alkoxy group; $R_3$ represents a hydrogen or halogen atom or a methyl or methoxy group; and $R_2$ represents a hydrogen or halogen atom, a $C_{1-8}$ alkyl or alkoxy group, or a phenyl or chlorophenoxy group.

12. 3,4-dimethoxy-4'chlorobenzophenone.

13. 3,4-dimethoxy-4'-phenylbenzophenone.

14. 3,4-dimethoxy-4'-(4"-chlorophenoxy)-benzophenone.

European Patent Office

# EUROPEAN SEARCH REPORT

Application number: EP 87202595.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US - A - 3 983 176 (OSAMU YAMADA et al.)<br>* Columns 1,2 *<br>-- | 1-3,8, 10 | C 07 C 49/84<br>C 07 C 45/68<br>C 07 C 149/32<br>C 07 C 147/06<br>C 07 C 147/14<br>C 07 C 143/86<br>C 07 C 107/06<br>C 07 C 79/36<br>C 07 C 97/10<br>C 07 D 295/10<br>C 07 C 103/76<br>C 07 C 121/76 |
| X | DE - A1 - 3 120 747 (CASSELLA)<br>* Claim 2; page 5, lines 6-9; page 6, lines 5-15 *<br>-- | 1-3,8 | |
| A | DE - A1 - 2 806 563 (HOECHST)<br>* Claim 1; examples 2,6,7 *<br>-- | 1,8 | |
| A | US - A - 4 124 726 (YASUHIKO HAMAZAKI et al.)<br>* Abstract; column 2, lines 27-68 *<br>-- | 1-3,8 | TECHNICAL FIELDS SEARCHED (Int. Cl.4) |
| A | DE - B - 1 139 111 (ROHM & HAAS)<br>* Claim 1 *<br>-- | 1,8 | C 07 C 49/00<br>C 07 C 45/00<br>C 07 C 149/00<br>C 07 C 147/00<br>C 07 C 143/00<br>C 07 C 107/00<br>C 07 C 79/00<br>C 07 C 103/00<br>C 07 C 97/00<br>C 07 D 295/00<br>C 07 C 121/00 |
| A | US - A - 3 907 837 (FRANZ EFFENBERGER et al.)<br>* Claims 1,2; examples 3,8-10 *<br>-- | 1,2,8 | |
| A | CESARE FERRI "Reaktionen der organischen Synthese", 1978<br>GEORG THIEME VERLAG, Stuttgart<br>* Pages 64, 314, 324 *<br>-- | 1-4,6, | |

The present search report has been drawn up for all claims

| Place of search | Date of completion of the search | Examiner |
|---|---|---|
| VIENNA | 22-03-1988 | REIF |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

Application number

EP 87202595.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE - A1 - 2 707 102 (SHIONOGI)<br>* Claims; examples 1,5-9,13, 14,24,26,28,29 *<br>---- | 1,6 | |
| | | | TECHNICAL FIELDS SEARCHED (Int. Cl.4) |

The present search report has been drawn up for all claims

| Place of search | Date of completion of the search | Examiner |
|---|---|---|
| VIENNA | 22-03-1988 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document